# EUROPEAN PATENT SPECIFICATION

(11) **EP 1 467 779 B1**
(45) Date of publication and mention of the grant of the patent: **18.02.2009**
(21) Application number: 02793748.1
(22) Date of filing: 27.12.2002
(51) Int. Cl.: A61M 1/00, A61M 5/00

(54) **MICRODIALYSIS PROBE WITH INSERTING MEANS AND ASSEMBLY**
MIKRODIALYSESONDE MIT INSERTIONSMITTEL UND ZUSAMMENBAU
ENSEMBLE ET SONDE DE MICRODIALYSE PRESENTANT DES MOYENS D'INSERTION

(30) Priority: 28.12.2001 SE 0104469
(43) Date of publication of application: 20.10.2004
(73) Proprietor: Microbiotech/SE AB, 100 74 Stockholm (SE)
(72) Inventor: Model, Per, 100 74 Stockholm (SE); Karlsson, Hans, 100 74 Stockholm (SE)
(74) Representative: Schneiders, Josef
(86) International application number: PCT/SE2002/002454
(87) International publication number: WO 2003/055540

(56) References cited:
- EP-A1- 0 401 179
- WO-A1-00/10464
- WO-A1-01/03752
- WO-A1-01/03763
- WO-A1-95/20983
- WO-A1-95/20991
- US-A- 4 090 502
- US-A- 5 106 365
- US-A- 5 353 792
- US-A- 5 372 582
- US-A- 5 607 390

## Description

### FIELD OF THE INVENTION

The present invention relates to a microdialysis probe and an assembly comprising a microdialysis probe and a cannula.

### BACKGROUND OF THE INVENTION

Microdialysis probes are used, for instance, in collecting tissue fluid samples from humans and animals for diagnostic purposes, either intermittently or continuously. A microdialysis probe comprises a semipermeable membrane, one face of which is in contact with tissue whereas its other face is in contact with a solvent flowing inside of the probe. The solvent which is water or saline or similar is adduced to the probe via a first tube and passes the membrane. Depending on the pore size of the membrane small and medium size molecules in the tissue fluid can penetrate the membrane and are carried away by the probe solvent, thus forming a sample solution. The sample solution is carried away from the probe by a second flexible tube or similar. The analytes in the sample solution can be analyzed in a conventional manner, either continuously or discontinuously. For instance, the sample solution is made to flow through a quartz cell for detection and/or measurement of analytes that absorb UV light of a certain wavelength.

There are some important restrictions of design in regard of microdialysis probes. In general, the diameter of a probes has to be small to allow it to be inserted into tissue by means of a cannula provided with a sharp tip.

The use of a cannula is due to the fragile nature of the microdialysis probe, in particular of its membrane. Upon insertion the cannula has to be withdrawn to expose the membrane to the tissue. The design of thin microdialysis cannulae known in the art and used in clinical practice however requires the use of a particular kind of cannula with a longitudinal slit, such as the cannula disclosed in WO 95/20991. The reason for this is that the proximal (rear) end portion of state-of-the-art cannulae is rather bulky due to the mounting of the solvent-adducing tube and the sample-removing at the probe. For instance, in the microdialysis probe of this design disclosed in WO 95/20983 said tubes are mounted at a distal end piece the diameter of which (in a transverse direction to the extension of the probe) is substantially greater than the diameter of the probe. The distal end piece thus is too large to allow a cannula of a physiologically acceptable diameter (about 1 mm or less) to be drawn over the end piece rearwards, and thus withdrawn. The microdialysis probe of WO 95/20983 thus necessitates the use of a cannula having a longitudinal slit, such as the cannula disclosed in WO 20991.

U.S. Patent No. 5,106,365 (Hernandez) discloses a microdialysis probe which is substantially rotationally symmetrical and comprises an outer sleeve at the distal end of which a dialysis membrane is fixed in such a manner so as to avoid extra thickness at the junction between the membrane and the sleeve. At the proximal end the sleeve is mounted in a second sleeve of substantially larger diameter. The tubes for delivery and removal of dialysis fluid are made of a stiff material. No technique for insertion of this known probe into tissue so as to avoid potential damage is disclosed.

WO 01/03752 A1 provides a microdialysis probe, the location of which maybe monitored and controlled using means such as X-rays or NMR during insertion and withdrawal or during dialysis in order to facilitate the placement of the probe at a predetermined location and to control the location of the probe.

The present invention seeks to avoid the aforementioned problems.

### OBJECTS OF THE INVENTION

It is an object of the present invention to provide a microdialysis probe which does not require the use of a cannula that needs to be split longitudinally for removal.

It is an object of the present invention to provide a microdialysis probe which can be easily produced.

It is another object of the present invention to provide an assembly of such microdialysis probe and a corresponding cannula.

Further objects of the invention are evident from the following short description of the invention, the attached drawings illustrating a preferred embodiment, the detailed description thereof, and the appended claims.

### SUMMARY OF THE INVENTION

The dialysis probe of the invention, like dialysis probes known in the art, has substantial longitudinal extension, that is, extension in a proximal/distal direction, whereas its extension in a transverse direction, that is, its diameter, is comparatively small.

According to the present invention is provided a microdialysis probe comprising a tubiform probe body, a tubiform dialysis membrane disposed distally of the probe body, wherein a rear fitting is connected to the probe body at the proximal end thereof so as to make a proximal end portion of the probe body to be enclosed by the rear fitting thereby forming a first chamber communicating via the probe body lumen with a second chamber formed by the membrane which is connected to the probe body at a distal end portion thereof, wherein the probe comprises flexible conduits for adducing and abducing dialysis fluid, one distal end portion of the conduits is disposed in the probe body and the remaining end portion of the other conduit is disposed in the rear fitting, the probe being, apart from the conduit portions disposed externally of the probe body, substantially rotationally symmetrical, wherein one of said conduits comprises an S-shaped portion inside of the probe adjacent to the distal end of the other conduit.

It is preferred for the conduits comprising the Shaped portion to be the abducing conduits and to have a distal end portion extending distally of the probe body. Preferably the sum of the outer diameters of the conduits corresponds to the inner diameter of the probe body. It is preferred for the probe to have a maximum outer diameter of 1.5 mm or less, in particular a diameter from 0.8 to 1.2 mm.

It is preferred to make a probe body distal end portion to be enclosed by a proximal end portion of the membrane. At its distal end the membrane is plugged or closed in any other convenient manner.

According to a first preferred aspect of the adducing and abducing flexible conduits are sealingly fastened at the rear fitting, in particular at a proximal end portion thereof, so as to make one of them extend through the rear fitting and the probe body and having its front end located in the front chamber adjacent to the distal end of the membrane, and to make the other of them having its front end disposed in the rear chamber. It is preferred for the first and second tubes to be sealingly fastened to the rear flitting at a proximal portion thereof.

According to a second preferred aspect of the invention it is preferred for the probe body to be radiologically opaque, in particular in regard of the other elements of the probe and the tissue to which it is intended to be inserted, such as fat or connective tissue. It is particularly preferred for the probe body to consist of a polymer or a mixture of polymers and radiologically opaque material finely dispersed therein, in particular barium sulphate.

According to a third preferred aspect of the invention is disclosed the combination of the microdialysis probe of the invention and a cannula the lumen of which is larger than the maximum outer diameter of the probe. It is also preferred for the microdialysis probe to be received in the cannula in its entirety except for proximal end portions of the adducing and abducing flexible tubes, and in combination with which it can be inserted into living tissue and from which it is slidingly removable in a proximal direction. It is preferred for the cannula to comprises a holding means disposed near its proximal end.

According to a fourth preferred aspect of the invention is disclosed the combination of the probe and the cannula in a configuration ready for insertion packaged in a sterile condition.

### SHORT DESCRIPTION OF THE FIGURES

The invention will now be explained in more detail by reference to a preferred but not limiting embodiment illustrated in a drawing showing in
- Fig. 1: a microdialysis probe according to the invention, in a side view;
- Fig. 2: the probe of Fig. 1, in a longitudinal section;
- Fig. 3: the probe of Figs. 1 and 2, provided with a cannula according to the invention for insertion into tissue, in the same view as in Fig. 1;
- Fig. 4: the probe of Fig. 1, in an enlarged transverse section A-A.

### DESCRIPTION OF A PREFERRED EMBODIMENT

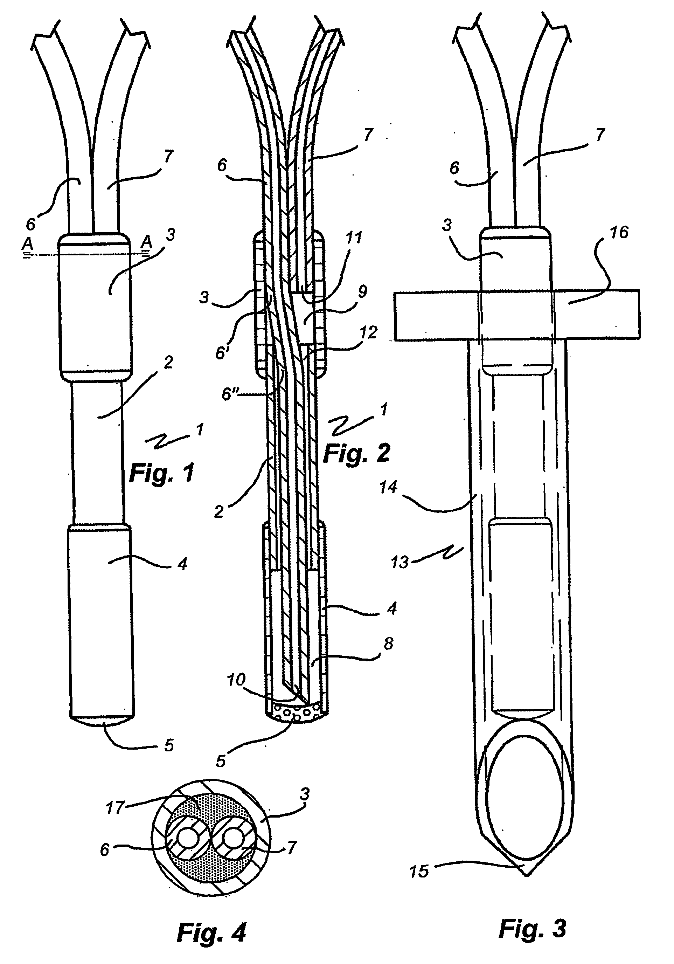

The microdialysis probe 1 of the invention shown in the Figures comprises a substantially rigid cylindrical probe body 2 to which a rear fitting (a proximal sleeve) 3 is attached by gluing 17 at the sealed rear opening of the rear fitting 3 protrude first and second flexible tubes 6, 7, the proximal sends of which are not shown. The distal end portion of the probe body 2 is covered by a dialysis membrane 4. The sealing gluing 17 at the rear opening of the rear fitting 3 is shown in Figure 4. It may be provided, for instance, by a two-component polyurethane glue. Similarly, the membrane 4 is sealed at its front end by a.polyurethane plug 5. The probe body 2 can be made of any suitable stiff but resilient plastic material, such as polyamide, but also of mental such as, for instance, stainless steel. The probe body of plastic material can be made opaque to radiation, such as X-rays, by incorporation of finely dispersed barium sulphate. The rear fitting 3 is made of polyimide. The flexible tubes 6 and 7 are made of fluorinated polyethylene/propene. The maximum width of the probe 2 (at the rear fitting 3) was determined to be 1.0 mm.

As shown in Fig. 2 the interior of the probe 1 comprises three communicating compartments: (a) a proximal chamber 9 defined by the rear fitting 3, its sealed rear opening and the probe body 2, (b) the interior of the probe body 2, (c) a distal chamber 8 defined by the membrane 4 and the membrane plug 5. The first flexible tube 6 for carrying away dialysis fluid extends through the proximal chamber 9 and the probe body 2 into the distal chamber 8; its front opening 10 is disposed adjacent to the membrane plug 5. The portion of the first flexible tube 6 disposed in the proximal chamber 9 and in close proximity thereof has an oblong S-formed configuration, with bends at 6' and 6". In other words, the first flexible tube 6 has two parallel (but not coaxial) sections, one of them located in the probe body 2, the other in the proximal portion of the rear fitting 3 through which the distal end portion of the second flexibles tube 7 extends; the portion of the flexible tube 6 extending between these parallel sections forms an oblique angle with the probe taxis. The lumen of the probe body 2 is slightly greater than the outer diameter of the first flexible tube 6 thus providing for communication between the proximal chamber 9 and the distal chamber 8 through the interstice 12 formed between the outer wall of the first flexible tube 6 and the inner wall of the cylindrical probe body 2. The second flexible tube 7 extends into the proximal chamber 9 where its distal opening 11 is disposed. Dialysis fluid adduced through the second flexible tube 7 emerges at its distal end 11 into the proximal chamber 9, flows from there through the interstice 12 and enters the distal chamber 8. During its passage through the distal chamber 8 the dialysis fluid takes up material, such as small and medium size molecules, from body fluid surrounding the probe 1 that diffused through the membrane 4; the technique can however also be used for simultaneous administration of drugs and other compounds dissolved in the dialysis fluid to the surrounding tissue. Finally the dialysis fluid loaded with said material and enters the distal opening 10 of the first flexible tube 6 by which it is emptied from the probe 1. Normally the dialysis fluid is a physiological aqueous sodium chloride solution. Cellulose acetate (manufactured by Akzo N.V., Netherlands), polyethersulphone and other semipermeable membranes of various kind and various cut-off pore sizes determining the size of molecules that can pass through the membrane 4 are known in the art and can be used in the probe 1 of the invention. The dialysis fluid loaded with those molecules is fed to an analysis unit (not shown) disposed downstream of the first flexible tube 6. The dialysis fluid is fed into the probe 1 via the second flexible tube 7 by pumping means (not shown) disposed upstream of the probe 1, such as, for instance, a fluid reservoir kept under a positive pressure, or a mini-roller pump at a selected feed rate, in particular at a selected constant feed rate.

The transverse dimensions of the probe 1 are generally kept as small permitted by considerations of mechanical stability, membrane area, and flow rate. The microdialysis probe 1 of the invention can be inserted into tissue by appropriate means, such as the cannula means disclosed in WO 95/20991. However, the design of the probe 1 of the invention allows the use of a cannula means that need not be split longitudinally for withdrawal. Such a cannula of the invention 13 is shown in Fig. 3. It is of simple design, comprising a stainless steel cannula body 14 having a pointed tip 15 at its distal end and a holder 16 attached near its proximal end. In Fig. 3 a combination of the microdialysis probe 1 of the invention and the cannula 13 of the invention is shown in state ready for insertion into living tissue, the distal portion of the probe 1 being disposed in the lumen of the cannula 13. Since the lumen of the cannula is wider than the widest portion of the probe 1, the rear fitting 3, the cannula 13 can be removed by withdrawal in a proximal direction upon insertion but before the proximal ends (not shown) of the flexible tubes 6,7 are attached to the dialysis pump (not shown) and the analysis unit (not shown), respectively. The use of a cannula for insertion is due to the limited mechanical stability of the microdialysis probe; however, under certain conditions, such as with tissue that offers little mechanical resistance, in particular neural tissue, the use of a cannula can he dispensed with.

## Claims

1. A microdialysis probe comprising a tubiform probe body (2), a tubiform dialysis membrane (4) disposed distally of the probe body (2), wherein a rear fitting (3) is connected to the probe body (2) at the proximal end thereof so as to make a proximal end portion of the probe body (2) to be enclosed by the rear fitting (3) thereby forming a first chamber (9) communicating via the probe body (2) lumen with a second chamber (8) formed by the membrane (4) which is connected to the probe body (2) at a distal end portion thereof, wherein the probe (1) comprises flexible conduits (6, 7) for adducing and abducing dialysis fluid, one distal end portion of the conduits is disposed in the probe body (2) and the remaining end portion of the other conduit is disposed in the rear fitting (3), the probe (1) being, apart from the conduit portions disposed externally of the probe body (2), substantially rotationally symmetrical, **characterized in that** one of said conduits (6) comprises an S-shaped portion inside of the probe (1) adjacent to the distal end of the other conduit.

2. The probe of claim 1, wherein the conduit (6) comprising the S-shaped portion is the abducing conduit and has a distal end portion extending distally of the probe body (2).

3. The probe of claim 1 or 2, wherein the sum of the outer diameters of the conduits (6, 7) corresponds to the inner diameter of the probe body (2).

4. The probe of any of claims 1-3 having a maximum outer diameter of 1.5 mm or less, in particular a diameter from 0.8 to 1.2 mm.

5. The probe of any of claims 1-4, wherein a probe body (2) distal end portion is enclosed by a proximal end portion of the membrane (4).

6. The probe of any of claims 1-5, wherein the membrane (4) is plugged end or closed in any other convenient manner at its front end.

7. The probe of any of the preceding claims, wherein the adducing and abducing flexible conduits (7, 6) are sealingly fastened at the rear fitting (3), in particular at a proximal end portion thereof, so as to make one of them extend through the rear fitting (3) and the probe body (2) and having its front end located in the front chamber (8) adjacent to the distal end of the membrane (4), and to make the other of them having its front end disposed in the rear chamber (9).

8. The probe of claim 7, wherein the adducing and abducing tubes (7, 6) are sealingly fastened to the rear fitting (3) at a proximal portion thereof.

9. The probe of any of claims 1-8, wherein the probe body (2) is radiologically opaque.

10. The probe of claim 9, wherein the probe body (2) consists of a polymer or a mixture of polymers and radiologically opaque material finely dispersed therein.

11. The probe of claim 10, wherein said radiologically opaque materials is barium sulphate.

12. The combination of the microdialysis probe (1) of any of claims 1-11 and a cannula (13) the lumen of which is larger than the maximum outer diameter of the probe (1).

13. The combination of claim 12, wherein the microdialysis probe (1) is designed to be received in the cannula (13) in its entirety except for proximal end portions of the adducing and abducing flexible tubes (7,6), and in combination with which it can be inserted into living tissue and from which it is slidingly removable in a proximal direction.

14. The combination of claim 12 or 13, wherein the cannula (13) comprises a holding means (16) disposed near its proximal end.

15. The combination of any of claims 12-14 in a configuration ready for insertion packaged in a sterile condition.

## Patentansprüche

1. Mikrodialysesonde, aufweisend einen rohrförmigen Sondenkörper (2) und eine rohrförmige, distal an dem Sondenkörper (2) angeordnete Dialysemembran (4), wobei ein hinteres Anschlussstück (3) mit dem proximalen Ende des Sondenkörpers (2) verbunden ist, so dass ein proximaler Endabschnitt des Sondenkörpers (2) von dem hinteren Anschlussstück (3) umschlossen ist, wodurch eine erste Kammer (9) gebildet wird, die über das Lumen des Sondenkörpers (2) mit einer zweiten Kammer (8) in Verbindung steht, die durch die Membran (4) gebildet ist, welche mit einem distalen Endabschnitt des Sondenkörpers (2) verbunden ist, wobei die Sonde (1) flexible Leitungen (6, 7) zum Zuführen und Abführen von Dialyseflüssigkeit aufweist, wobei ein distaler Endabschnitt der Leitungen in dem Sondenkörper (2) und der andere Endabschnitt der anderen Leitung in dem hinteren Anschlussstück (3) angeordnet ist, wobei die Sonde (1) abgesehen von den außerhalb des Sondenkörpers (2) angeordneten Leitungsabschnitten im Wesentlichen rotationssymmetrisch ist, **dadurch gekennzeichnet, dass** eine der Leitungen (6) in der Sonde (1) nahe des distalen Endes der anderen Leitung einen S-förmigen Abschnitt aufweist.

2. Sonde nach Anspruch 1, wobei die Leitung (6) mit dem S-förmigen Abschnitt die Abführleitung ist und einen distalen Endabschnitt hat, der sich distal von dem Sondenkörper (2) erstreckt.

3. Sonde nach Anspruch 1 oder 2, wobei die Summe der Außendurchmesser der Leitungen (6, 7) dem Innendurchmesser des Sondenkörpers (2) entspricht.

4. Sonde nach einem der Ansprüche 1 bis 3, aufweisend einen maximalen Außendurchmesser von 1,5 mm oder weniger, insbesondere einen Durchmesser von 0,8 bis 1,2 mm.

5. Sonde nach einem der Ansprüche 1 bis 4, wobei ein distaler Endabschnitt des Sondenkörpers (2) von einem proximalen Endabschnitt der Membran (4) umschlossen ist.

6. Sonde nach einem der Ansprüche 1 bis 5, wobei die Membran (4) an ihrem vorderen Ende verstopft oder auf eine irgendeine andere zweckmäßige Art und Weise verschlossen ist.

7. Sonde nach einem der vorhergehenden Ansprüche, wobei die zuführenden und abführenden flexiblen Leitungen (6, 7) abdichtend an dem hinteren Anschlussstück (3), insbesondere an einem proximalen Endabschnitt desselben, befestigt sind, um zu erreichen, dass eine von ihnen sich durch das hintere Anschlussstück (3) und den Sondenkörper (2) erstreckt und ihr vorderes Ende in der vorderen Kammer (8) in der Nähe des distalen Endes der Membran (4) angeordnet hat, und dass die andere von ihnen ihr vorderes Ende in der hinteren Kammer (9) angeordnet hat.

8. Sonde nach Anspruch 7, wobei die zuführenden und abführenden Rohre (6, 7) abdichtend an einem proximalen Abschnitt des hinteren Anschlussstücks (3) befestigt sind.

9. Sonde nach einem der Ansprüche 1 bis 8, wobei der Sondenkörper (2) radiologisch undurchsichtig ist.

10. Sonde nach Anspruch 9, wobei der Sondenkörper (2) aus einem Polymer oder einer Mischung von Polymeren und einem darin fein verteilten, radiologisch undurchsichtigen Material besteht.

11. Sonde nach Anspruch 10, wobei das radiologisch undurchsichtige Material Bariumsulfat ist.

12. Kombination der Mikrodialysesonde (1) nach einem der Ansprüche 1 bis 11 mit einer Kanüle (13), deren Lumen größer als der maximale Außendurchmesser der Sonde (1) ist.

13. Kombination nach Anspruch 12, wobei die Mikrodialysesonde (1) ausgestaltet ist, um bis auf proximale Endabschnitte der zuführenden und abführenden flexiblen Rohre (6, 7) vollständig in der Kanüle (16) aufgenommen zu werden, und in Kombination mit dieser in lebendes Gewebe eingeführt und von diesem gleitend in proximaler Richtung entfernt werden kann.

14. Kombination nach Anspruch 12 oder 13, wobei die Kanüle (13) ein Haltemittel (16) aufweist, das in der Nähe ihres proximalen Endes angeordnet ist.

15. Kombination nach einem der Ansprüche 12 bis 14 in einer zur Einführung fertigen, steril verpackten Anordnung.

## Revendications

1. Sonde de microdialyse comprenant un corps de sonde tubulaire **(2),** une membrane de dialyse tubulaire **(4)** placée de manière distale par rapport au corps de sonde **(2),** dans laquelle une monture arrière **(3)** est raccordée au corps de sonde **(2)** au niveau de son extrémité proximale afin qu'une partie d'extrémité proximale du corps de sonde **(2)** soit enfermée par la monture arrière **(3),** formant ainsi une première chambre **(9)** communiquant via l'ouverture du corps de sonde **(2)** avec une deuxième chambre **(8)** formée par la membrane **(4)** qui est raccordée au corps de sonde **(2)** au niveau d'une partie distale de celui-ci, dans laquelle la sonde **(1)** comprend des conduits flexibles **(6, 7)** pour amener et évacuer un fluide de dialyse, une partie d'extrémité distale des conduits est placée dans le corps de sonde **(2)** et la partie d'extrémité restante de l'autre conduit est placée dans la monture arrière **(3),** la sonde **(1)** étant, en dehors des parties de conduit placées à l'extérieur du corps de sonde **(2),** sensiblement symétrique par rotation, **caractérisée en ce que** l'un desdits conduits **(6)** comprend une partie en forme de S à l'intérieur de la sonde **(1)** adjacente à l'extrémité distale de l'autre conduit.

2. Sonde selon la revendication 1, dans laquelle le conduit **(6)** comprenant la partie en forme de S est le conduit d'évacuation et possède une partie d'extrémité distale s'étendant de manière distale par rapport au corps de sonde **(2).**

3. Sonde selon la revendication 1 ou 2, dans laquelle la somme des diamètres extérieurs des conduits **(6, 7)** correspond au diamètre intérieur du corps de sonde **(2).**

4. Sonde selon l'une des revendications 1 à 3, ayant un diamètre extérieur maximal de 1,5 mm ou moins, en particulier un diamètre de 0,8 à 1,2 mm.

5. Sonde selon l'une des revendications 1 à 4, dans laquelle une partie d'extrémité distale du corps de sonde (2) est enserrée par une partie d'extrémité proximale de la membrane **(4).**

6. Sonde selon l'une des revendications 1 à 5, dans laquelle la membrane **(4)** est à extrémité bouchée ou est fermée de tout autre manière appropriée au niveau de son extrémité antérieure.

7. Sonde selon l'une des revendications précédentes, dans laquelle les conduits flexibles d'alimentation et d'évacuation **(7, 6)** sont fixés hermétiquement au niveau de la monture arrière **(3),** en particulier au niveau d'une partie d'extrémité proximale, afin que l'un d'entre eux s'étende à travers la monture arrière **(3)** et le corps de sonde (2) et qu'ils aient leur extrémité antérieure située dans la chambre antérieure **(8)** adjacente à l'extrémité distale de la membrane **(4),** et l'autre d'entre eux ait son extrémité antérieure placée dans la chambre postérieure **(9).**

8. Sonde selon la revendication 7, dans laquelle les tubes d'alimentation et d'évacuation **(7, 6)** sont fixés hermétiquement à la monture arrière **(3)** au niveau d'une partie proximale de celle-ci.

9. Sonde selon l'une des revendications 1 à 8, dans laquelle le corps de sonde **(2)** est radiologiquement opaque.

10. Sonde selon la revendication 9, dans laquelle le corps de sonde **(2)** se compose d'un polymère ou d'un mélange de polymère et d'un matériau radiologiquement opaque finement dispersé dedans.

11. Sonde selon la revendication 10, dans laquelle ledit matériau radiologiquement opaque est le sulfate de baryum.

12. Combinaison d'une sonde de microdialyse **(1)** selon l'une des revendications 1 à 11 et d'une canule **(13)** dont l'ouverture est plus grande que le diamètre extérieur maximal de la sonde **(1).**

13. Combinaison selon la revendication 12, dans laquelle la sonde de microdialyse **(1)** est conçue pour être logée dans la canule **(13)** dans son intégralité à l'exception de parties d'extrémité proximales des tubes flexibles **(7, 6)** d'alimentation et d'évacuation, et en combinaison avec lesquels elle peut être insérée dans un tissu vivant et depuis lequel elle peut être retirée par coulissement dans une direction proximale.

14. Combinaison selon la revendication 12 ou 13, dans laquelle la canule **(13)** comprend un moyen de préhension (16) placé près de son extrémité proximale.

15. Combinaison selon l'une des revendications 12 à 14, dans une configuration prête à être insérée et emballée dans un état stérile.
